# FASCICULE DE BREVET EUROPEEN

(11) **EP 0 725 594 B1**
(45) Date de publication et mention de la délivrance du brevet: **16.06.1999**
(21) Numéro de dépôt: 95900171.0
(22) Date de dépôt: 27.10.1994
(51) Int. Cl.: A61B 5/117

(54) **PROCEDE DE RELEVE D'EMPREINTES DIGITALES**
VERFAHREN ZUM AUFNEHMEN VON FINGERABDRÜCKEN
FINGERPRINTING RECORDING METHOD

(30) Priorité: 29.10.1993 FR 9313103
(43) Date de publication de la demande: 14.08.1996
(73) Titulaire: COMMUNAUTE ECONOMIQUE EUROPEENNE (CEE), 2920 Luxembourg (LU)
(72) Inventeur: HAESSLER, Auguste, F-67810 Holtzheim (FR); SOWINSKA, Malgorzata, F-67117 Ittenheim (FR); NOIRARD, Pierre, F-68300 St-Louis (FR); MIEHE, Joseph-Albert, F-67200 Strasbourg (FR)
(74) Mandataire: Nuss, Pierre
(86) Numéro de dépôt international: FR9401256
(87) Numéro de publication internationale: WO9511625

(56) Documents cités:
- EP-A- 0 045 913
- WO-A-88/00024
- WO-A-88/01730
- WO-A-93/19433
- US-A- 4 176 205
- US-A- 4 794 260
- US-A- 4 983 846

## Description

La présente invention concerne les techniques d'identification et de mise en évidence d'indices ou de preuves exploitables et juridiquement valables, notamment par la prise en compte de traces caractéristiques laissées par les individus, et a pour objet un procédé de relevé d'empreintes digitales, ainsi qu'un dispositif pour la mise en oeuvre et l'utilisation d'un révélateur spécifique à cet effet.

Actuellement, les empreintes digitales sont généralement, en vue de leur exploitation, photographiées après révélation par dépôt d'une poudre adaptée, dont la couleur, la nature et la composition dépendent des caractéristiques (couleur, texture, indice de réflexion, etc.) du support matériel portant l'empreinte, nécessitant l'apport constant d'une pluralité de poudres différentes sur les lieux des relevés.

En outre, la technique des poudres précitée est mise en défaut dans de nombreux cas, notamment lorsque les supports sont polychromes, imprimés, rugueux ou brillants, aboutissant à des relevés non exploitables.

De plus, cette technique nécessite souvent, en vue de tenter d'obtenir des résultats exploitables, le recours à un prélèvement de l'empreinte à l'aide d'un film adhésif ou à un traitement chimique préalable du support (chèque par exemple). Ces interventions supplémentaires détériorent ou déforment forcément les empreintes qui y sont soumises, augmentant ainsi les difficultés de lecture et d'analyse de ces dernières, voire rendant ces opérations impossibles à réaliser.

Il est également connu une méthode de relevé d'empreintes basée sur l'excitation d'une poudre d'un colorant (substance fluorescente) par un faisceau de forte énergie laser et la prise de vue de l'empreinte fluorescente résultante (cf. US-A-4 794 260).

Toutefois, cette méthode, bien que, dans certains cas, plus performante que la précédente, est, du fait du matériel encombrant nécessaire, difficile à mettre en oeuvre in situ, et peut donner lieu à l'observation d'artefacts.

La présente invention a notamment pour but de pallier l'ensemble des inconvénients précités et de permettre d'obtenir des résultats d'une qualité nettement supérieure à ceux obtenus avec l'état des techniques exposées ci-dessus, ce même pour des supports non exploitables par les techniques précitées.

A cet effet la présente invention a pour objet un procédé de relevé d'empreintes digitales sur tout support matériel, présentant les caractéristiques décrites dans la revendication 1.

L'invention sera mieux comprise grâce à la description ci-après, qui se rapporte à un mode de réalisation préféré, donné à titre d'exemple non limitatif, et expliqué avec référence aux dessins schématiques annexés, dans lesquels:
la figure 1 est une vue schématique du dispositif conforme à l'invention;
la figure 2A représente l'image d'une empreinte peu visible sur un chèque sous éclairage naturel;
la figure 2B représente l'image de l'empreinte de la figure 2A relevée au moyen du procédé conforme à l'invention;
les figures 2C et 2D représentent respectivement l'image de l'empreinte de la figure 2B après deux traitements numériques distincts complémentaires;
la figure 3A représente l'image d'une empreinte peu visible sur un billet de banque sous éclairage naturel;
la figure 3B représente l'image de l'empreinte de la figure 3A relevée au moyen du procédé conforme à l'invention;
les figures 3C et 3D représentent respectivement l'image de l'empreinte de la figure 3B après deux traitements numériques distincts complémentaires;
la figure 4A représente l'image d'une empreinte peu visible sur un emballage plastique d'un quotidien délivré par routage, sous éclairage naturel;
la figure 4B représente l'image de l'empreinte de la figure 4A relevée au moyen du procédé conforme à l'invention après augmentation du contraste dans les zones sous-exposées;
les figures 4C et 4D représentent respectivement l'image de l'empreinte de la figure 4B après deux traitements numériques distincts complémentaires;
les figures 5A à 5D représentent les images d'une empreinte à cheval sur l'étiquette et le corps en matière plastique transparent d'une bouteille remplie de liquide, respectivement sous éclairage naturel (5A), telle que relevée par l'intermédiaire du procédé conforme à l'invention (5B) et telle qu'elle se présente après deux traitements numériques distincts complémentaires de l'image de la figure 5B (5C et 5D);
les figures 6A à 6D représentent les images d'une empreinte sur un manche en bois, respectivement, sous éclairage naturel (6A), telle que relevée par l'intermédiaire du procédé conforme à l'invention (6B) et telle qu'elle se présente après deux traitements numériques distincts et complémentaires de l'image de la figure 6B (6C et 6D);
les figures 7A à 7D représentent les images d'une empreinte sur une publicité polychrome sur support papier, respectivement, sous éclairage naturel (7A), telle que relevée au moyen du procédé conforme à l'invention (7B) et telle qu'elle se présente après deux traitements numériques distincts et complémentaires de l'image de la figure 7B (7C et 7D);
les figures 8A à 8D représentent les images d'une empreinte présente sur un manche en bois, identique à celui de la figure 6A, respectivement, sous éclairage naturel (8A), telle que relevée par l'intermédiaire de la technique de fluorescence laser de l'art antérieur (8B) et telle qu'elle se présente (8C et 8D) après deux traitements numériques distincts similaires à ceux ayant aboutis aux figures 6C et 6D, et,
les figures 9A à 9D représentent les images d'une empreinte présente sur une publicité polychrome similaire à celle de la figure 7A, respectivement, sous éclairage naturel (9A), telle que relevée par l'intermédiaire de la technique de fluorescence laser de l'art antérieur (9B) et telle qu'elle se présente (9C et 9D) après deux traitements numériques distincts similaires à ceux ayant aboutis aux figures 7C et 7D.

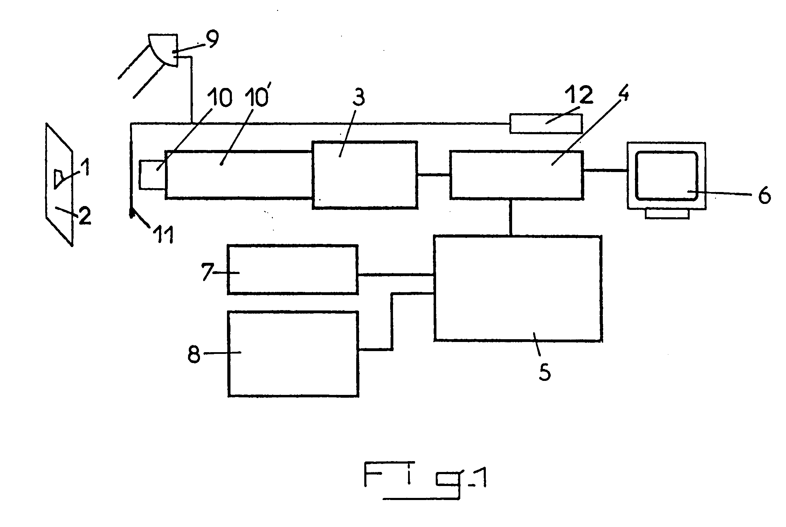

Conformément à l'invention, et comme le montre en partie la figure 1 des dessins annexés, le procédé de relevé d'empreintes digitales consiste essentiellement, quel que soit le support matériel 2 portant l'empreinte 1, à appliquer sur l'empreinte 1 à relever une poudre révélatrice à propriétés phosphorescentes, à soumettre l'empreinte 1 à un éclairage adéquat, puis à créer une zone d'obscurité au moins au niveau de la portion de support 2 portant l'empreinte 1, à effectuer une ou plusieurs prises de vue de l'empreinte 1 dans l'obscurité et, enfin, à visualiser, à archiver, à traiter, à éditer, à transmettre et/ou à analyser les images résultant desdites prises de vue.

Selon une première caractéristique de l'invention, le procédé précité peut consister avantageusement à réaliser les prises de vue au moyen d'une caméra 3 numérique très sensible, préférentiellement du type dispositif à transfert de charges (coupled charge device CCD), et à transférer les images acquises vers une unité de traitement numérique 4 pourvu de moyens de stockage 5 et connecté à des dispositifs de visualisation 6, d'édition, d'impression 7 et/ou d'interface 8 avec des réseaux commutés ou hertziens de télécommunication, en vue de la transmission des images numériques acquises au moyen de ladite caméra numérique 3.

Afin d'augmenter la netteté et le contraste par rapport au fond constitué par le support 2, de l'empreinte 1 à relever, il est indispensable d'utiliser une poudre ou un pigment phosphorescent(e) à grains fins, dont l'émission maximale, après excitation par une lumière blanche ou naturelle, se situe dans une plage de longueurs d'onde correspondant à la plage de sensibilité maximale de la caméra CCD 3 mise en oeuvre, les images finales étant obtenues par sommation d'un nombre variable de prises de vues successives de l'empreinte phosphorescente dans l'obscurité.

La poudre phosphorescente précitée peut consister par exemple, en un pigment phosphorescent à grains fins du type de celui connu sous la référence M1070 par la société MAIER GmbH & Co. KG (Allemagne).

L'application de ce pigment sur l'empreinte 1 à relever pourra être effectuée selon les méthodes classiques connues de l'homme du métier, à savoir saupoudrage de l'empreinte 1, puis élimination de l'excès de pigment phosphorescent révélateur.

La portion de support 2 portant l'empreinte 1 recouverte de pigment révélateur est préférentiellement soumise, en vue de son excitation, à un éclairage en lumière blanche, fourni par une lampe halogène 9, par exemple, mais peut également être exposée à la lumière ambiante.

Une prise de vue à l'exposition précitée, consécutive de l'empreinte 1 dans l'obscurité permet, du fait de la phosphorescence de la poudre ou du pigment révélateur excité, de faire ressortir et d'isoler l'empreinte 1 par rapport à son support 2. L'image de l'empreinte 1 relevée est alors totalement indépendante des caractéristiques et des propriétés (nature, couleur, rugosité, texture, réflectivité, brillance, etc.) de la portion de support 1 qui la porte, ces dernières n'influençant pas la netteté de l'image finale de l'empreinte 1 qui présente un excellent contraste.

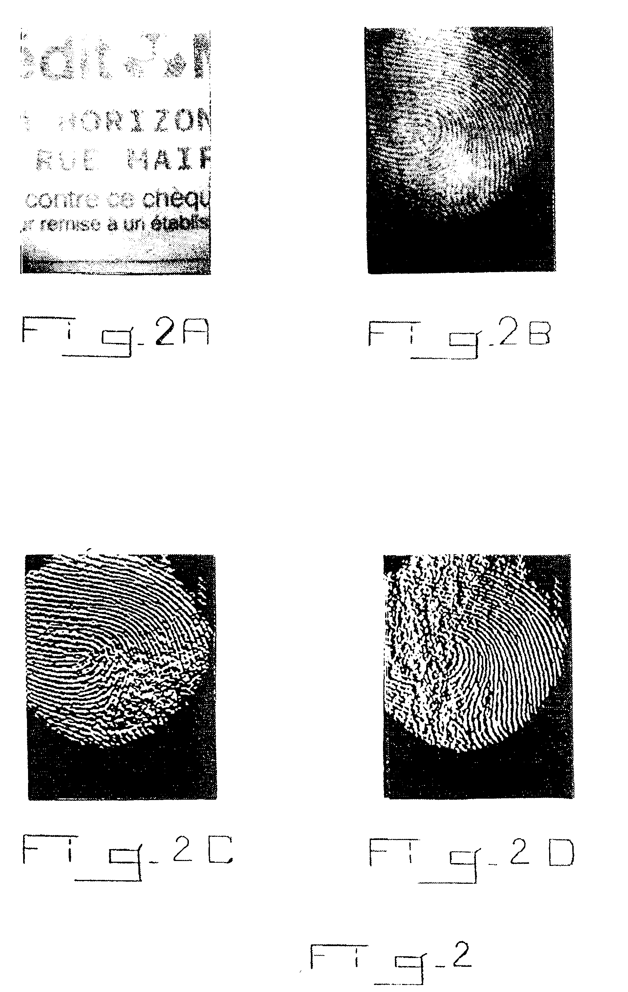

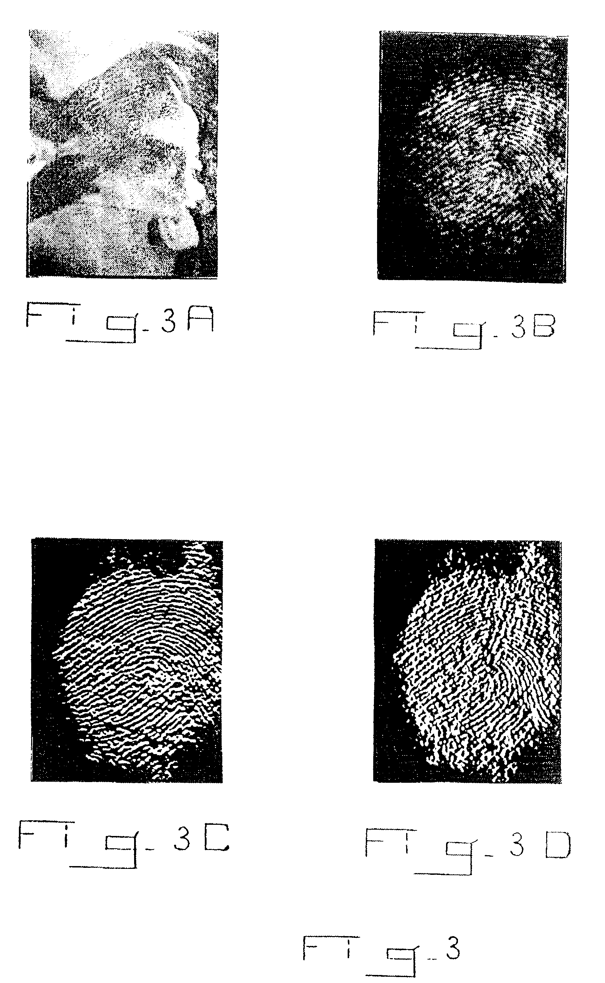

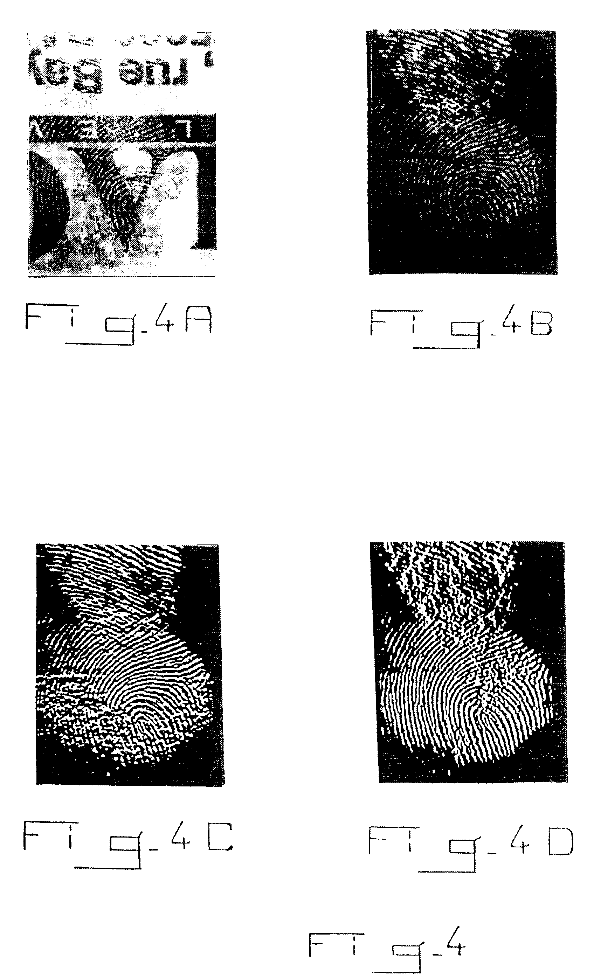

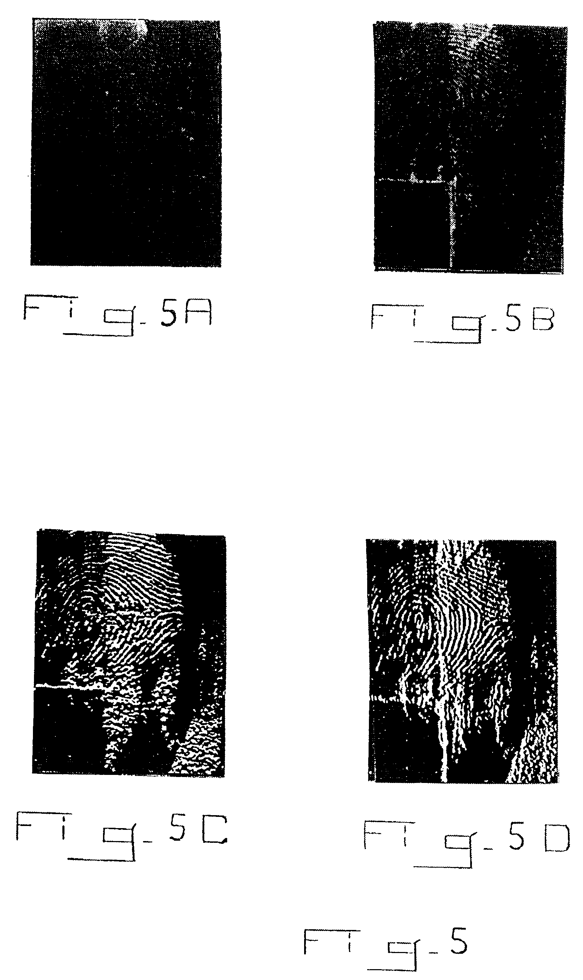

En outre, l'obtention d'une image numérique au moyen d'une caméra CCD 3 à haute résolution et très sensible et par accumulation d'une pluralité de prises de vue identiques (plusieurs centaines), augmente considérablement la netteté et le contraste du motif de l'empreinte 1 par rapport au support (comparer notamment les figures 2A, 3A, 4A, 5A, 6A et 7A aux figures 2B, 3B, 4B, 5B, 6B et 7B respectivement).

De plus, la durée d'acquisition extrêmement courte desdites prises de vues successives (quelques secondes), permet de profiter de la période du maximum d'émissivité dudit pigment phosphorescent révélateur, située immédiatement après établissement de l'obscurité.

Contrairement aux procédés photographiques la mise en oeuvre d'une caméra CCD 3 permet, du fait de l'absence de développement, la visualisation immédiate de l'image acquise, en vue notamment de son contrôle avant archivage, exploitation ou transmission et, il peut être prévu alors d'effectuer, le cas échéant, soit l'acquisition d'un nouvelle image de l'empreinte 1 obtenue avec des paramètres, tels que nombre total de prises de vue à sommer, netteté, contraste, distance, temps de pose par prise de vue ou encore durée et intensité de l'excitation, modifiés, soit un traitement numérique de l'image acquise, ou d'une portion au moins de cette dernière, en vue d'augmenter sa qualité et la netteté du motif de l'empreinte et de ses caractéristiques distinctives. Il est important de noter que ces traitements sont effectués sur un relevé d'empreinte, en l'absence de l'image du support 2.

Les figures 1 à 7 désignées par C et D représentent les images des empreintes des figures correspondantes désignées par B, après binarisation suivant l'axe X (figures C) et après binarisation suivant l'axe Y (figures D), ces deux traitements améliorant des zones distinctes et complémentaires du motif de l'empreinte relevée.

Selon une autre caractéristique de l'invention, le procédé consiste à effectuer également une prise de vue de l'empreinte 1 sous éclairage normal, dont l'image résultante sera associée à l'image de l'empreinte 1 obtenue dans l'obscurité, différents textes ou signes phosphorescents informatifs pouvant également être ajoutés au moment de la prise de vue des images numériques enregistrées avant codage, archivage, édition, impression et/ou transmission.

Ainsi les images des figures 2A à 9A servent de preuve de la présence de l'empreinte 1 sur le support 2 donné et les images des figures 2B à 9B constituent les documents de travail pour l'identification.

Le procédé selon l'invention peut être réalisé au moyen d'un dispositif décrit ci-après et principalement constitue, d'une part, par un moyen 3 de prises de vues numériques, d'autre part, par une unité 4 de traitement numérique, par exemple du type micro-ordinateur, pour l'acquisition, l'analyse, le transfert, l'archivage et/ou le traitement des images délivrées par ledit moyen 3 de prises de vue numériques et, enfin, par des dispositifs de visualisation 6, de commande, d'édition, d'impression 7 et/ou de transmission 8 associés à ladite unité 4 de traitement numérique (Figure 1).

Le moyen de prise de vues numériques se présente préférentiellement sous la forme d'une caméra numérique du type à couplage de charge (CCD), pourvue d'une optique adaptée 10 ainsi que d'un intensificateur de lumière 10'.

L'unité 4 de traitement numérique comportera les différentes cartes et logiciels nécessaires à l'acquisition des images numériques délivrées par la caméra CCD 3, à leur archivage dans des mémoires associées 5, à leur visualisation sur un écran 6 avec différentes palettes de couleurs et histogrammes associés, à leur édition sur des supports informatiques amovibles, à leur impression sur papier par des imprimantes 7 et à leur transfert par des réseaux commutés numériques vers un centre d'identification au moyen d'une interface 8 adaptée.

De plus, des logiciels spécifiques de traitement d'images numériques ou d'aide à la prise de vue pourront également être mis en oeuvre par l'unité 4 à la demande de l'utilisateur, permettant, par exemple, d'éviter les surexpositions ou sous-expositions dues aux variations spatiales de l'intensité de la phosphorescence, l'acquisition desdites images numériques étant, en outre, pratiquement indépendante du temps de pose (variable de manière continue).

En outre, afin de faciliter la mise en oeuvre du procédé décrit ci-dessus et de réaliser les prises de vue dans la période d'émission maximale de la poudre ou du pigment phosphorescent(e), il peut être prévu des dispositifs d'éclairage 9 et de mise en obscurité 11 de la portion au moins de support 2 portant l'empreinte 1 à relever, pouvant être actionnés au moyen d'une commande unique 12 en synchronisme avec le moyen 3 de prises de vues numériques.

Il convient de noter que, bien que décrit plus particulièrement en rapport avec un procédé mettant en oeuvre un dispositif de prises de vues et de traitement d'images du type numérique, la poudre phosphorescente décrite ci-dessus, constituant un révélateur d'empreintes universel, peut également être utilisée, indépendamment dudit procédé, dans le cadre des méthodes "à poudre révélatrice" traditionnelles (par exemple par prélèvement de l'empreinte au moyen d'un film adhésif), en tant que révélateur unique pour relever des empreintes digitales 1 sur tout support 2 matériel en lumière naturelle.

Grâce à l'invention, il est donc possible de réaliser un procédé simple de relevé d'empreintes digitales 1, ne mettant en oeuvre qu'un unique révélateur quel que soit la nature et les caractéristiques du support 2 et permettant d'effectuer des relevés exploitables, de bonne qualité, sur de nombreux supports difficilement exploitables ou non étudiés actuellement par l'intermédiaire des méthodes connues (surfaces polychromes, bois, métaux et surfaces brillantes, films ou objet en un matériau plastique, zone de jonction entre deux supports de natures différentes, etc.), ce sans aucun traitement particulier de l'empreinte 1 ou du support 2, susceptible de détériorer ces derniers.

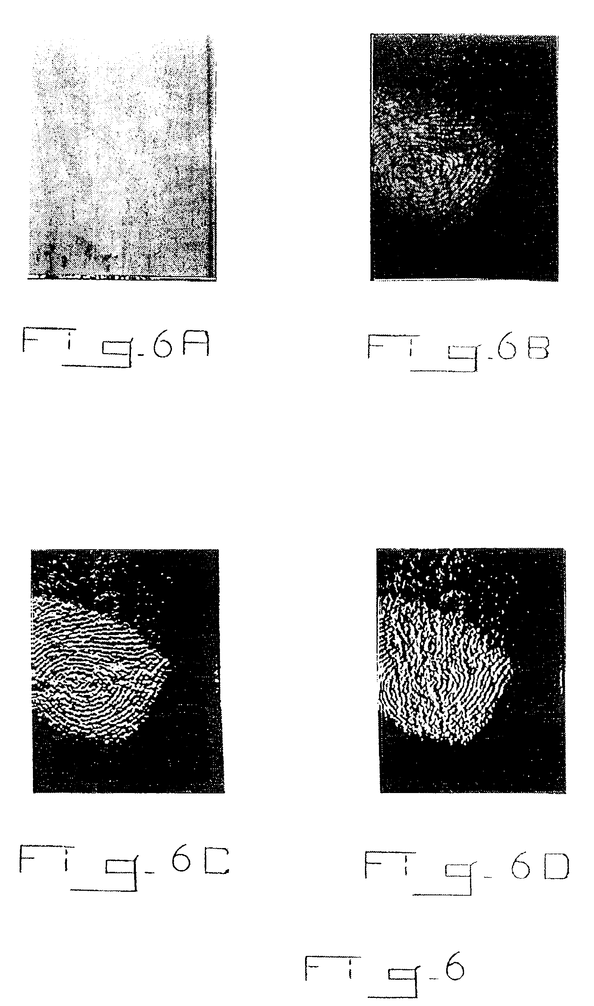

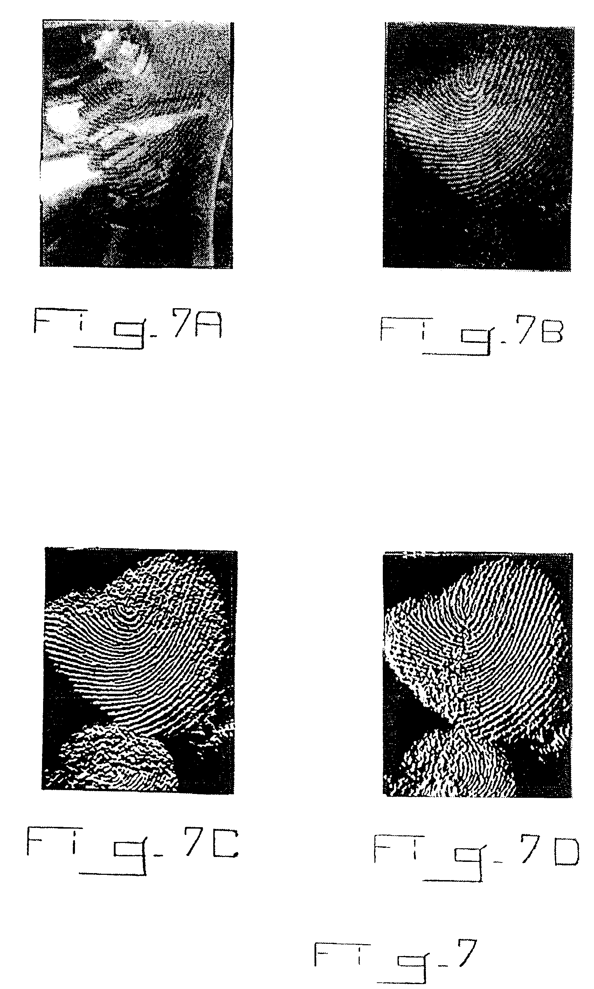

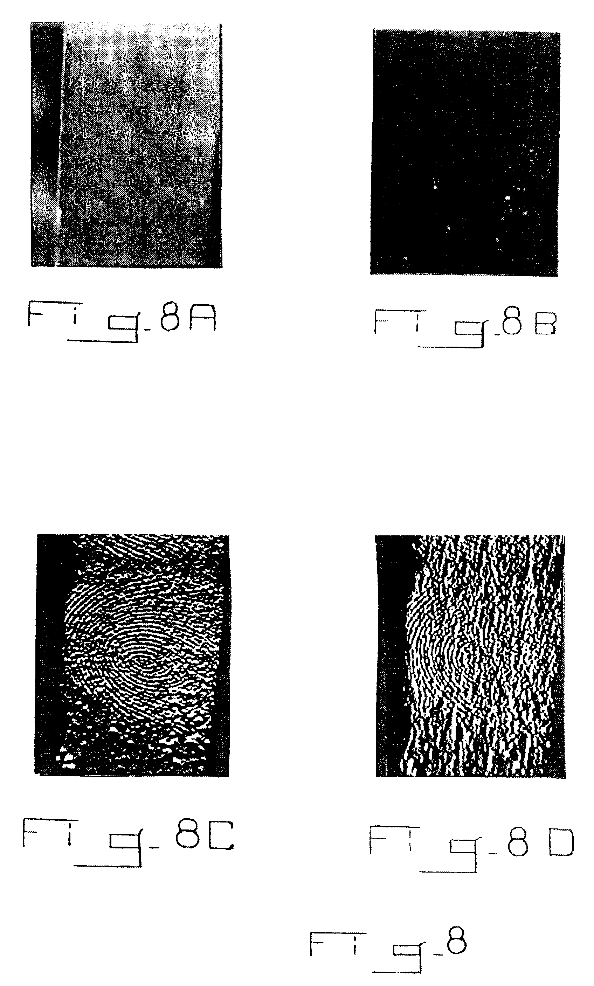

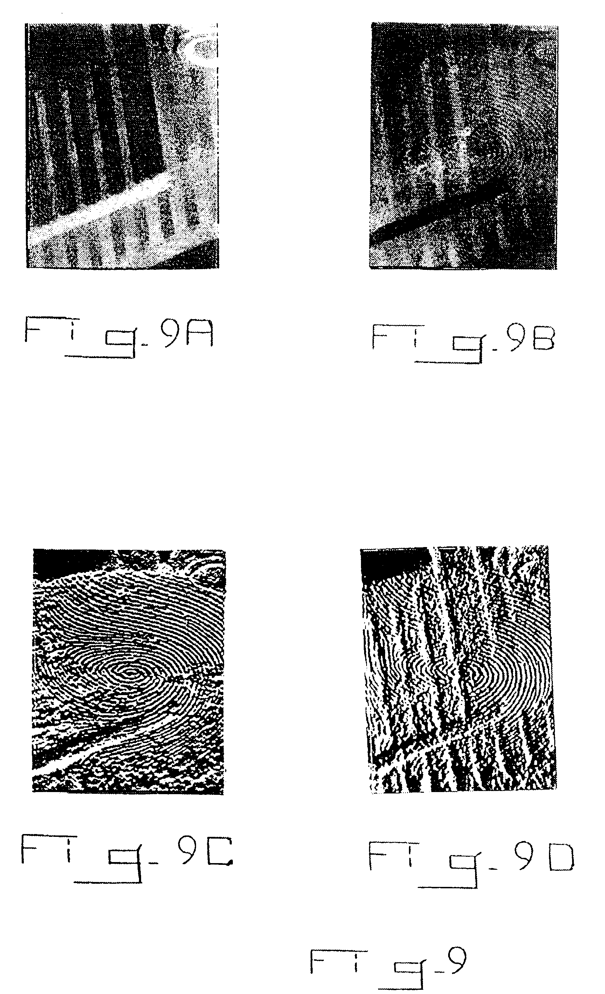

La mise en oeuvre de ce révélateur unique, aboutissant à des images d'empreintes à très haut contraste par rapport au support 2 (comparer les figures 6B et 7B aux figures 8B et 9B par exemple) avec une excitation simple et inoffensive, en combinaison avec un moyen 3 de prises de vues numériques directes, ayant une résolution et une sensibilité élevées, autorise une exploitation en temps réel et in situ des relevés effectués, avec possibilité d'optimisation instantanée des prises de vues et de manipulations numériques multiples des images obtenues.

Par ailleurs, l'ensemble du dispositif est relativement compact et facilement transportable.

Bien entendu, l'invention n'est pas limitée au mode de réalisation décrit et représenté aux dessins annexés. Des modifications restent possibles, notamment du point de vue de la constitution des divers éléments, ou par substitution d'équivalents techniques, sans sortir pour autant du domaine de protection de l'invention.

## Revendications

1. Procédé de relevé d'empreintes digitales sur tout support matériel, caractérisé en ce qu'il consiste quel que soit le support (2), à appliquer sur l'empreinte (1) à relever une poudre révélatrice à propriétés phosphorescentes, à soumettre l'empreinte à un éclairage adéquat, puis à créer une zone d'obscurité au moins au niveau de la portion de support (2) portant l'empreinte (1), à effectuer plusieurs prises de vue de l'empreinte (1) dans l'obscurité en vue d'obtenir une image numérique par accumulation d'une pluralité de prises de vue identiques et, enfin, à visualiser, à archiver, à traiter, à éditer, à transmettre et/ou à analyser les images résultant desdites prises de vue.

2. Procédé selon la revendication 1, caractérisé en ce qu'il consiste à réaliser les prises de vue au moyen d'une caméra (3) numérique très sensible, préférentiellement du type à couplage de charge (CCD), et à transférer les images acquises vers une unité de traitement numérique (4) pourvu de moyens de stockage (5) et connecté à des dispositifs de visualisation (6), d'édition, d'impression (7) et/ou d'interface (8) avec des réseaux commutés ou hertziens de télécommunication.

3. Procédé selon la revendication 2, caractérisé en ce qu'il consiste à utiliser une poudre ou un pigment phosphorescent(e) à grains fins dont l'émission maximale, après excitation par une lumière blanche, se situe dans une plage de longueurs d'onde correspondant à la plage de sensibilité maximale de la caméra CCD (3) mise en oeuvre, les images finales étant obtenues par sommation d'un nombre variable de prises de vue successives.

4. Procédé selon l'une quelconque des revendications 2 et 3, caractérisé en ce qu'il consiste à visualiser, en vue de son contrôle, l'image numérique acquise avant archivage, exploitation par analyse et/ou transmission, et à effectuer, le cas échéant, soit l'acquisition d'une nouvelle image de l'empreinte (1) obtenue avec des paramètres, tels que nombre total de prises de vue à sommer, netteté, contraste, distance, temps de pose par prise de vue ou encore durée et intensité de l'excitation, modifiés, soit un traitement numérique de l'image acquise, ou d'une portion au moins de cette dernière, en vue d'augmenter sa qualité et la netteté du motif de l'empreinte (1) et de ses caractéristiques distinctives.

5. Procédé selon l'une quelconque des revendications 1 à 4, caractérisé en ce qu'il consiste à effectuer également une prise de vue de l'empreinte (1) sous éclairage normal, dont l'image résultante est associée à l'image de l'empreinte (1) obtenue dans l'obscurité, différents textes ou signes informatifs pouvant également être ajoutés au moment de la prise de vue des images numériques enregistrées avant codage, archivage, édition, impression et/ou transmission.

6. Procédé selon l'une quelconque des revendications 1 à 5, caractérisé en ce que les différentes opérations sont réalisées au moyen d'un dispositif constitué, d'une part, par un moyen (3) de prises de vues numériques se présentant sous la forme d'une caméra numérique du type à couplage de charge (CCD) pourvue d'une optique adaptée (10) ainsi que d'un intensificateur de lumière (10'), d'autre part, par une unité (4) de traitement numérique du type micro-ordinateur, pour l'acquisition, l'analyse, le transfert, l'archivage et/ou le traitement des images délivrées par ledit moyen (3) de prises de vue numériques et, enfin, par des dispositifs de visualisation (6), de commande, d'édition, d'impression (7) et/ou de transmission (8) associés à ladite unité (4) de traitement numérique, ledit dispositif comportant, en outre, des dispositifs d'éclairage (9) et de mise en obscurité (11) de la portion au moins de support (2) portant l'empreinte (1) à relever, pouvant être actionnés au moyen d'une commande unique (12) en synchronisme avec le moyen (3) de prises de vues numériques.

7. Utilisation d'une poudre à propriétés phosphorescentes en tant que révélateur unique pour relever des empreintes digitales (1) sur tout support (2) matériel en lumière naturelle, par prises de vues successives de l'empreinte phosphorescente dans l'obscurité.

## Claims

1. Method of taking fingerprints on any material substrate, characterised in that it involves applying a developer powder with phosphorescent properties onto the fingerprint (1) on any substrate (2), adequately illuminating the fingerprint, forming a darkened region at least over the portion of the substrate (2) which bears the fingerprint (1), taking one or more pictures of the darkened fingerprint (1) in order to obtain a digital image by accumulation of a plurality of identical shots and finally displaying, filing, processing, editing, transmitting and/or scanning the resultant images.

2. Method according to claim 1, characterised in that it involves taking pictures using a very sensitive digital camera (3), preferably of the charge-coupled (CCD) type and transferring the images acquired to a digital processing unit (4) provided with storage means (5) and connected to devices for displaying (6), editing, printing (7) and/or interfacing (8) with switched or radio-frequency telecommunications networks.

3. Method according to claim 2, characterised in that it involves using a phosphorescent powder or pigment having fine grains of which the maximum emission, after excitation by a white light, lies within a wavelength range corresponding to the maximum sensitivity range of the CCD camera (3) used, the final images being obtained by summation of a variable number of successive shots.

4. Method according to any one of claims 2 and 3, characterised in that it involves displaying, for the control thereof, the digital image acquired prior to filing, handling by scanning and/or transmission and effecting, if applicable, either the acquisition of a new image of the fingerprint (1) obtained with parameters such as the total number of shots to be added, definition, contrast, distance, setting time per shot or again duration and intensity of excitation modified, or alternatively digital processing of the acquired image or of at least a portion thereof in order to increase its quality and the definition of the pattern of the fingerprint (1) and of its distinctive characteristics.

5. Method according to any one of claims 1 to 4, characterised in that it involves also taking pictures of the fingerprint (1) under normal illumination, the resultant image of which is associated with the image of the fingerprint (1) obtained in darkness, wherein various informative texts or signs can also be added at the moment of taking pictures of the digital images recorded prior to coding, filing, editing, printing and/or transmission.

6. Method according to any one of claims 1 to 5, characterised in that the various operations are carried out using a device consisting, on the one hand, of a digital picture-taking means (3) in the form of a digital camera of the charge-coupled (CCD) type provided with an adapted lens (10) and with a light intensifier (10'), on the other hand of a digital processing unit (4) of the microcomputer type for the acquisition, scanning, transfer, filing and/or processing of the images delivered by said digital picture-taking means (3) and, finally, of display (6), control, editing, printing (7) and/or transmitting (8) devices associated with said digital processing unit (4), said device also comprising illuminating devices (9) and darkening devices (11) at least for the portion of the substrate (2) bearing the fingerprint (1) to be recorded, which can be actuated using a single control means (12) synchronised with the digital picture-taking means (3).

7. Use of a powder having phosphorescent properties as a single developer for taking fingerprints (1) on any material substrate (2) in natural light by taking successive pictures of the phosphorescent fingerprint in darkness.

## Patentansprüche

1. Verfahren zum Aufnehmen von auf einem Träger aufgeprägten Fingerabdrücken,
dadurch gekennzeichnet,
daß es folgende Schritte aufweist:
- ein Entwicklerpulver mit phosphoreszierenden Eigenschaften wird auf den Fingerabdruck (1) eines beliebigen Trägers (2) gegeben,
- der Fingerabdruck wird angemessen beleuchtet,
- dann wird eine Dunkelzone erzeugt, die mindestens denjenigen Teil des Trägers (2) umfaßt, der den Fingerabdruck (1) trägt,
- mehrere Aufnahmen werden vom abgedunkelten Fingerabdruck (1) gemacht, und zwar um ein numerisches Bild durch Sammeln einer Vielzahl von identischen Aufnahmen zu erhalten, und
- die sich aus diesen Aufnahmen ergebenden Bilder werden schließlich sichtbar gemacht, archiviert, verarbeitet, ausgegeben, übertragen und/oder analysiert.

2. Verfahren nach Anspruch 1,
dadurch gekennzeichnet,
daß die Aufnahmen mittels einer sehr empfindlichen, numerischen Kamera (3), insbesondere einer ladungsgekoppelten Kamera (CCD), gemacht werden und daß die so erhaltenen Bilder zu einer numerischen Verarbeitungseinheit (4) übertragen werden, die mit einem Speicher (5) und mit einem Sichtgerät (6) verbunden ist und mit einer Ausgabeeinrichtung, einer Druckeinrichtung (7) und/oder einer Adaptereinrichtung (8) in Verbindung steht, die Zugang zu Wechselstrom- oder Fernsprechfrequenznetzen hat.

3. Verfahren nach Anspruch 2,
dadurch gekennzeichnet,
daß ein phosphoreszierendes Pulver oder Pigment aus feinen Körnern verwendet wird, dessen maximale Emission nach Erregung durch weißes Licht in einem Wellenlängenbereich liegt, das dem Bereich maximaler Empfindlichkeit der betriebenen Kamera CCD (3) entspricht, wobei die endgültigen Bilder durch Summierung einer veränderlichen Anzahl von aufeinanderfolgenden Aufnahmen gewonnen werden.

4. Verfahren nach Anspruch 2 oder 3,
dadurch gekennzeichnet,
daß das vor der Archivierung und der Auswertung durch Analyse und/oder Übertragung gewonnene, numerische Bild für seine Kontrolle sichtbar gemacht wird und daß gegebenenfalls entweder die Erlangung eines neuen Bilds des Fingerabdrucks (1) mit veränderlichen Parametern bewirkt wird, die die Gesamtzahl der zu summierenden Aufnahmen, die Bildschärfe, den Kontrast, den Zeitabstand, die Belichtungszeit für die Aufnahme und auch die Dauer und die Stärke der Erregung betreffen, oder eine numerische Verarbeitung des gewonnenen Bilds oder mindestens eines Teils dieses Bildes bewirkt wird, um die Qualität und die Motivschärfe des Fingerabdrucks (1) und bestimmte Bildeigenschaften zu verbessern.

5. Verfahren nach einem der Ansprüche 1 bis 4,
dadurch gekennzeichnet,
daß zusätzlich eine Aufnahme des Fingerabdrucks (1) bei Normalbeleuchtung gemacht wird, dessen gewonnenes Bild dem bei Dunkelheit gewonnenen Bild des Fingerabdrucks (1) zugeordnet ist, wobei verschiedene informierende Texte oder Zeichen im Moment der Aufnahme der numerischen Bilder zugefügt werden können, bevor diese codiert, archiviert, ausgegeben, gedruckt und/oder übertragen werden.

6. Verfahren nach einem der Ansprüche 1 bis 5,
dadurch gekennzeichnet,
daß die verschiedenen Vorgänge mit einer Einrichtung durchgeführt werden, die folgendes aufweist:
- einerseits ein Mittel (3) für numerische Aufnahmen, das als numerische Kamera vom ladungsgekoppelten Typ (CCD) ausgebildet ist, die mit einer an einen Lichtverstärker (10') angepaßten Optik (10) versehen ist,
- andererseits eine als Mikrorechner ausgebildete Einheit (4) zur numerischen Verarbeitung für die Gewinnung, Analyse, Übertragung, Archivierung und/oder Verarbeitung von denjenigen Bildern, die vom Mittel (3) für numerische Aufnahmen abgegeben werden,
und daß die verschiedenen Vorgänge schließlich mit einer Sichteinrichtung (6), einer Befehlseinrichtung, einer Ausgabeeinrichtung, einer Druckeinrichtung (7) und/oder
einer Übertragungseinrichtung (8) durchgeführt werden, die alle mit der Einheit (4) zur numerischen Verarbeitung verbunden sind, wobei die eingangs genannte Einrichtung ferner eine Beleuchtungseinrichtung (9) und eine Abdunkelungseinrichtung (11) aufweist, die mindestens denjenigen Teil des Trägers (2) abdunkelt, der den aufzunehmenden Fingerabdruck trägt, wobei die letztgenannten Einrichtungen (9, 11) durch ein einzelnes Befehlsmittel (12) gesteuert sein können, das synchron mit dem Mittel (3) für numerische Aufnahmen betrieben wird.

7. Verwendung eines Pulvers mit phosphoreszierenden Eigenschaften als einmaliger Entwickler zum Aufnehmen von Fingerabdrücken (1), die insbesondere auf einem natürlich beleuchteten Träger (2) geprägt sind, für aufeinanderfolgende Aufnahmen des in Dunkelheit phosphoreszierenden Fingerabdrucks.
